# EUROPEAN PATENT APPLICATION

(11) **EP 1 362 612 A1**
(43) Date of publication of application: **19.11.2003**
(21) Application number: 02425310.6
(22) Date of filing: 16.05.2002
(51) Int. Cl.: A61M 25/06

(54) **Safety device for catheter guide needle**

(71) Applicant: Restelli, Sergio, 00100 Rome (IT); Righi, Nardino, 20047 Brugherio (Milano) (IT); Rossi, Roberto, 20151 Milano (IT)
(72) Inventor: Restelli, Sergio, 00100 Rome (IT); Righi, Nardino, 20047 Brugherio (Milano) (IT); Rossi, Roberto, 20151 Milano (IT)
(74) Representative: Petruzziello, Aldo

(57) **Abstract**

In a catheter (1) comprising a body (10) with an axial channel (11) communicating with a sheath or cannula (12) and a guide needle (3) that can be inserted, through the channel (11) of the body, into the sheath to guide it during insertion into the patient's body for administration of medicinal fluids, a safety device (100) is provided comprising a first sleeve (101) mounted so as to slide axially on the body (10) of the catheter and a second sleeve (104), integral with a needle-carrier (20) supporting the guide needle (3), mounted so as to slide axially on the first sleeve (102), the first and second sleeve being able to pass from a forward use position, wherein the second sleeve is situated over the first sleeve and the first sleeve is situated over the catheter body, to a retracted safety position, wherein the first sleeve protrudes rearward from the catheter body and the second sleeve protrudes rearward from the first sleeve, wherein the guide needle (3) is protected by the catheter body (1), by the first sleeve (101) and by the second sleeve (104).

## Description

The present invention refers to a safety device for a catheter guide needle or a needle-cannula for injection and administration of medicinal fluids into a cavity of a patient's body.

As is known the catheter, as well as for draining physiological fluids from the patient's body, also serves for injection or administration of medicinal fluids into the patient's body. For this purpose, a catheter generally comprises a central body connected to a flexible tube or sheath, commonly called a cannula, designed to be inserted into the patient's body for administration of medicinal fluids. Connectors are provided in the body of the catheter for connection of the catheter to bottles of medicinal fluids or to other medical instruments, such as pumps for administration of and therapy with medicinal fluids.

The body of the catheter also has an aperture with an axial channel through which a guide needle is inserted which extends inside the catheter sheath to guide it inside the patient's body. Once the catheter has been used, the tip of the guide needle remains outside the catheter body or the entire guide needle is extracted from the catheter body to be taken for disposal.

It is obvious that this operation of extraction of the guide needle proves dangerous, with the risk of injury to the user and to the personnel responsible for disposal. Furthermore, even in the event of the guide needle remaining inserted in the catheter, it must be considered that the tip of the guide needle protrudes forward from the catheter body and in any case this leads to the risk of injury or accidental needle sticks.

An object of the present invention is to overcome the drawbacks of the prior art by providing a safety device for a catheter guide needle that is able to avoid the danger and risk of accidental needle sticks.

Another object of the present invention is to provide such a safety device for a catheter guide needle that is practical and simple for the user to employ.

Yet another object of the present invention is to provide such a safety device for a catheter that is versatile and suitable to be applied in different types of catheters and guide needles existing on the market.

Yet another object of the present invention is to provide such a safety device for a catheter guide needle that is economical and simple to make.

These objects are achieved according to the invention with the characteristics listed in appended independent claim 1.

Advantageous embodiments of the invention are apparent from the dependent claims.

The safety device according to the invention is applied to a guide needle of a catheter. The catheter comprises a body with an axial channel communicating with a sheath or tube for administration of fluids. The guide needle can be inserted into the sheath through the channel of the catheter body, to guide it during insertion into the body of a patient to allow administration of medicinal liquids.

The safety device according to the invention comprises a first sleeve mounted so as to slide axially on the body of the catheter to pass from a forward use position, wherein it is situated over the catheter body to a retracted safety position, wherein it protrudes rearward from the catheter body.

The safety device further comprises a second sleeve, integral with a needle-carrier supporting the guide needle. The second sleeve is mounted so as to slide axially on the first sleeve to pass from a forward use position, wherein it is situated over the body of the first sleeve, to a retracted safety position, wherein it protrudes rearward from the first sleeve.

In this manner, when the two sleeves are in the forward use position, the guide needle is inserted in the sheath and protrudes forward from the catheter body and when the two sleeves are in the retracted safety position, the guide needle is protected by the catheter body, by the first sleeve and by the second sleeve.

The advantages of the safety device for a catheter guide needle or needle-cannula according to the invention are evident. In fact, once administration of the medicinal fluids has been carried out, said safety device allows the guide needle to be protected inside the body of the catheter and the two sleeves, thus avoiding the risk of accidental needle sticks by the user.

Further characteristics of the invention will be made clearer by the detailed description that follows, referring to a purely exemplary and therefore non-limiting embodiment thereof, illustrated in the appended drawings, wherein:
Figure 1 is an axial sectional view, illustrating in an exploded view a catheter, the various elements of the safety device and a catheter needle guide;
Figure 2 is a top plan view from above of the catheter with the safety device of Figure 1, assembled and in position ready for use;
Figure 3 is an axial sectional view along the sectional plane III-III of Figure 2;
Figure 4 is a top plan view from above of the catheter with the safety device, assembled, in the safety position;
Figure 5 is a side view of the catheter with the safety device, assembled, in the safety position shown in Figure 4;
Figure 6 is an axial sectional view along the sectional plane VI-VI of Figure 4.

A safety device for a catheter guide needle is described with the aid of the figures.

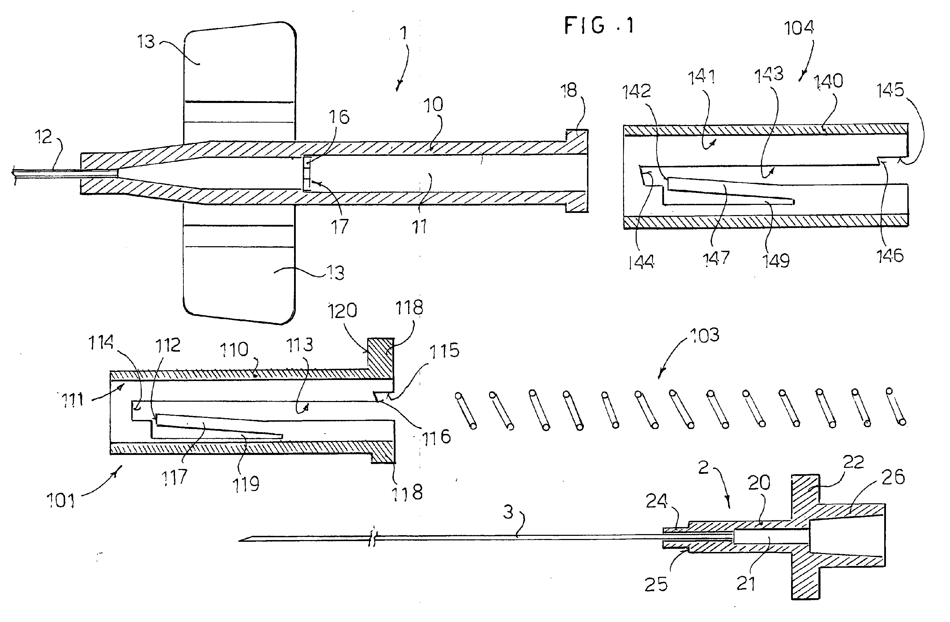

With reference in particular to Figure 1, a catheter, denoted as a whole with reference numeral 1, comprises a substantially cylindrical body 10, hollow on the inside so as to define an axial channel 11 open at the front and rear.

At the head of the catheter body 10, there is mounted a sheath or cannula, in the form of a flexible tube 12, hollow on the inside and communicating at least with the front portion of the axial channel 11, for administration of medicinal fluids.

Two flexible tongues 13 shaped like butterfly wings protrude radially from the body 10, in diametrically opposite positions and can be bent through gripping by the operator from a divergent position (shown in the figure) to a convergent position.

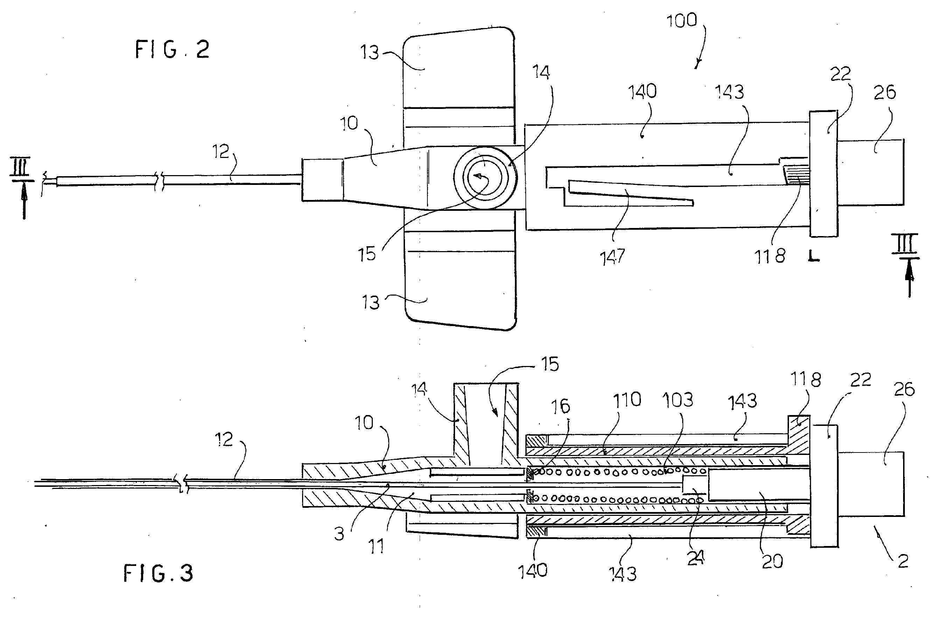

As shown in Figures 2 and 3, the body 10 of the catheter also comprises a connector 14 which protrudes radially therefrom and has a duct 15 communicating with the axial channel 11. The radial connector 14 is designed to be connected to a medical device for administration of a medicinal fluid for therapy. Said radial connector 14 is shown free and outwardly open, however before use it can be closed by means of a safety stopper.

Returning to Figure 1, the body 10 of the catheter has, in a central position, a collar or annular seal 16 that protrudes radially inward, dividing the axial channel 11 into a front chamber and a rear chamber. In this manner the seal 16 defines a radial abutment surface 17 at the rear.

Two nibs or tongues 18 that protrude radially outward in diametrically opposite positions are provided at the rear end of the body 10 of the catheter. The nibs 18 have an abutment surface 19 at the front.

A guide needle 3, shown broken off in Figure 1, is supported integrally by a needle-carrier 2. The needle-carrier 2 comprises a substantially cylindrical body 20 hollow on the inside, defining an axial channel 21. The body 20 of the guide needle has a disc-shaped flange 22 that protrudes radially therefrom.

A tang 24 with a smaller diameter than the body, so as to define an annular abutment surface 25, is provided in the fore end of the body 20 of the needle-carrier. Behind the flange 22, the body of the needle-carrier has a tapered or frusto conical connector 26 for connection of medical accessories of the catheter for introduction of medicinal fluids.

The guide needle 3 is inserted from the rear into the axial channel 11 of the body of the catheter to enter into the sheath 12 and guide it during insertion into the patient's body for administration of medicinal fluids. In this manner, a first medicinal fluid can be injected from the rear connector 26, through the guide needle 3, into the patient's body, and a second medicinal fluid can be injected from the radial connector 14 of the catheter body, through the cannula 12, into the patient's body, passing peripherally around the outer surface of the guide needle 3.

The safety device for a catheter guide needle, denoted as a whole with reference numeral 100, comprises a first sleeve 101, a second sleeve 104 and a coil spring 103.

The first sleeve 101 has a substantially cylindrical body 110, hollow on the inside so as to define an axial chamber 111 open at the front and rear. The inside diameter of the body 110 of the first sleeve is slightly greater than the outside diameter of the body 10 of the catheter, so that the first sleeve 101 can slide axially on the body of the catheter 1.

Two longitudinal slots 113 disposed in diametrically opposite positions are provided in the side wall of the body of the first sleeve. Each longitudinal slot 113 is open at the rear and closed at the front by means of an end-of-stroke abutment surface 114. Each longitudinal slot 113 has, at its rear end, a notch 115 defined by an oblique abutment surface 116. The longitudinal slot 113 with the notch 115 has a substantially L-shaped configuration.

In each longitudinal slot 113 there is provided an elastic tongue 117 which has a front abutment end 112 which opposes the front abutment 114 of the slot 113. Each flexible tongue 117 is defined by a longitudinal notch 119 in the side wall of the body 110 of the first sleeve, so as to be able to bend inside the longitudinal slot 113.

Each longitudinal slot 113 is able to receive the respective nib 18 of the rear end of the catheter body. In this manner the axial movement of the first sleeve 101 on the catheter body is guided by the nibs 18 which slide inside the longitudinal slots 113 that act as guides. The nibs 18 are blocked in the notches 115 of the longitudinal slots 113 in a bayonet coupling relationship.

It should be noted that the two nibs 18 protrude radially from the body 10 of the catheter for a distance equal to or smaller than the thickness of the body 110 of the first sleeve, so as not to protrude outward therefrom.

At the rear end of the body 110 of the first sleeve there are two nibs 118 and 118' which protrude radially therefrom in radially opposite directions. The two nibs 118 and 118' are disposed at an angular distance of about 90° with respect to the longitudinal slots 113. It should be noted that the nib 118 protrudes radially for a greater distance than the nib 118'. Each nib 118 and 118' has a transversally oblique front abutment surface 120.

The second sleeve 104 has a substantially cylindrical body 140, hollow on the inside so as to define an axial chamber 141 open at the front and rear. The inside diameter of the body 140 of the second sleeve is slightly greater than the outside diameter of the body 110 of the first sleeve, so that the second sleeve 104 can slide axially on the first sleeve 101.

In the side wall of the body 140 of the second sleeve there are two longitudinal slots 143 disposed in diametrically opposite positions and substantially similar to the longitudinal slots 113 of the first sleeve 101. Each longitudinal slot 143 is open at the rear and closed at the front by means of an end-of-stroke abutment 144. Each longitudinal slot 143 has in its rear end a notch 145 defined by an oblique abutment surface 146. The longitudinal slot 143 with the notch 145 has a substantially L-shaped configuration.

In each longitudinal slot 143 an elastic tongue 147 is provided which has a front abutment end 142 that opposes the front abutment 144 of the slot 143. Each flexible tongue 147 is defined by a longitudinal notch 149 in the side wall of the body 140 of the second sleeve, so as to be able to bend inside the longitudinal slot 143.

Each longitudinal slot 143 of the second sleeve is able to receive the respective nib 118 and 118' of the rear end of the body of the first sleeve 101. In this manner the axial movement of the second sleeve 104 on the body of the first sleeve 101 is guided by the nibs 118 and 118' which slide within the longitudinal slots 143 that act as guides. The nibs 118 and 118' are blocked in the notches 145 of the slots 143 in a bayonet coupling relationship.

It should be noted that the nib 118 protrudes radially from the body 110 of the first sleeve by a length greater than the thickness of the body 140 of the second sleeve. Thus, the nib 118 protrudes radially outward from the body of the second sleeve 104 so as to be able to be operated manually by the user. In this manner the nib 118 acts as an operating lever.

Assembly of the safety device 100, the catheter 1 and the relative guide needle 3 with the needle-carrier 2 will now be described.

Initially the first sleeve 101 is inserted from the rear into the second sleeve 104 so that the nibs 118, 118' of the first sleeve are locked in the notches 145 of the second sleeve 104 and the oblique abutment surfaces 120 of the nibs 118, 118' go into abutment against the oblique abutment surfaces 146 of the notches 145 of the longitudinal slots of the second sleeve.

The spring 103 is disposed around the front tang 24 of the needle-carrier 2 with one end of the spring 103 in abutment against the annular abutment surface 25 of the needle-carrier 2.

The second sleeve 104 is made integral with the needle-carrier 2, for example the rear end edge of the second sleeve 104 is fixed to the front surface of the flange 22 of the needle-carrier. Clearly, the second sleeve 104 can be made in one piece with the needle-carrier 2 and the first sleeve 101 is subsequently inserted from the front part of the second sleeve 104, so that the nibs 118 and 118' are locked inside the notches 146 of the second sleeve.

The assembly consisting of the two sleeves 101 and 104 and the needle-carrier 2 integral with the second sleeve 104 is inserted on the body 10 of the catheter. To be precise, the nibs 18 of the catheter body slide in the longitudinal guide slots 113 of the first sleeve until they are locked inside the notches 115 of the first sleeve. The front wall 19 of each nib 18 thus abuts against the oblique wall 116 of the notch 115 of the first sleeve.

In this situation, as shown in Figure 3, the spring 103 is compressed with one end in abutment against the annular abutment surface 25 of the needle-carrier and the other end in abutment against the annular seal collar 16 of the catheter body. The guide needle 3 extends axially through the entire channel 11 of the catheter body, enters the sheath 12 and acts as a guide for the sheath 12. It should be noted that the annular seal 16 prevents any liquid contained in the front chamber of the channel 11 of the catheter body from going into the rear chamber wherein the spring 103 and the operating mechanisms of the safety device 100 are situated.

In this situation, as shown in Figures 2 and 3, the catheter 1 is ready for use with the guide needle protruding forward from the body 10 of the catheter inside the sheath 12. Furthermore, it should be noted that the longest nib 118 protrudes outward from the body of the second sleeve 104 to be able to operated by the user.

Operation of the safety device 100 for the guide needle of a catheter according to the invention will now be described.

Once the catheter has been used for administration of a medicinal fluid, the user manually moves the operating nib 118 making it retract and subsequently rotate in a circumferential direction so as to cause the first sleeve 101 to rotate slightly with respect to its axis. As a result, the oblique abutment surfaces 120 of the nibs 118 and 118' disengage from the oblique abutment surfaces 146 of the second sleeve 104.

At the same time, rotation of the first sleeve 101 disengages the abutment surfaces 19 of the nibs 18 of the catheter body from the oblique abutment surfaces 116 of the first sleeve.

As a result, the first sleeve 101 is no longer retained by the nibs 18 of the catheter body 10 and at the same time the second sleeve 104 is no longer retained by the nibs 118, 118' of the first sleeve 101. Thus, through the action of the spring 103 which is released, the second sleeve 104, integral with the needle-carrier 20, retracts telescopically on the first sleeve 101 and at the same time the first sleeve 101 retracts telescopically on the catheter body 10.

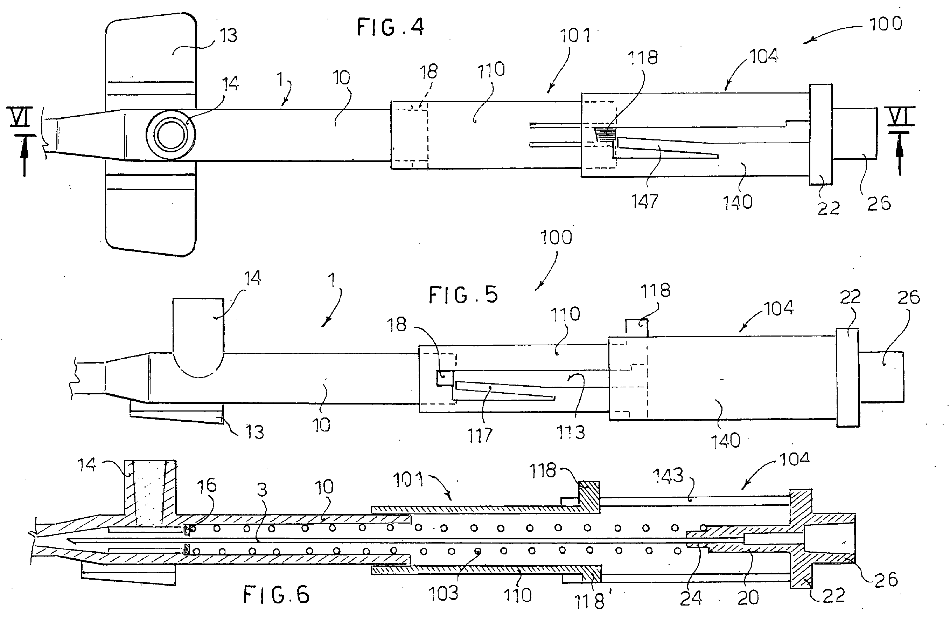

At the end of this action, as shown in Figures 4, 5 and 6, the needle 11, which is supported by the needle-carrier 20 integral with the second sleeve 104, is in a protected position, covered respectively by the catheter body 10, the first sleeve 101 and the second sleeve 104.

It should be noted that the telescopic movement of retraction of the second sleeve 104 on the first sleeve 101 is guided by the nibs 118, 118' of the first sleeve which slide in the guide slots 143 of the second sleeve, until the nibs 118, 118' bend the elastic tongues 147 and abut against the forward abutment surface 114 of the second sleeve, causing re-entry of the elastic tongues 147. The telescopic movement of retraction of the first sleeve 101 on the catheter body 1, on the other hand, is guided by the nibs 18 of the catheter body which slide in the guide slots 113 of the first sleeve, until the nibs 18 cause bending of the elastic tongues 117 and abut against the forward abutment surface 114 of the first sleeve.

It should further be noted that when the needle 3 is in the safety position, any axial movement of the second sleeve is prevented, since the nibs 118, 118' of the first sleeve are retained between the front abutment surface 144 of the slot of the second sleeve and the front abutment surface 142 of the elastic tongue of the second sleeve. At the same time, any axial movement of the first sleeve is prevented, since the nibs 18 of the catheter body are retained between the forward abutment surface 114 of the slot of the first sleeve and the forward abutment surface 112 of the elastic tongue of the first sleeve.

Numerous changes and modifications of detail within the reach of a person skilled in the art can be made to the present embodiment of the invention, without thereby departing from the scope of the invention expressed in the appended claims.

## Claims

1. A safety device for a guide needle of a catheter or needle-cannula, the catheter (1) comprising a body (10) with an axial channel (11) communicating with a sheath or cannula (12) for administration of fluids, the guide needle (3) being insertable, through the channel (11) of the body, inside the sheath (12) to guide it during insertion into the body of a patient, **characterised in that** said safety device (100) comprises
- a first sleeve (101) mounted so as to slide axially on said catheter body (10) to pass from a forward use position, wherein it is situated over the catheter body, to a retracted safety position, wherein it protrudes rearward from said catheter body, and
- a second sleeve (104), integral with a needle-carrier (20) supporting said guide needle (3), mounted to slide axially on said first sleeve (101) to pass from a forward use position, wherein it is situated over the body of the first sleeve, to a retracted safety position, wherein it protrudes rearward from said first sleeve, and said guide needle (3) is protected by the catheter body, by the first sleeve (101) and by the second sleeve (104).

2. A safety device (100) according to claim 1, **characterised in that** said body (10) of the catheter is coupled inside said first sleeve (101) and said first sleeve is coupled inside said second sleeve (104) in a telescopic coupling relationship.

3. A safety device (100) according to claim 1 or 2, **characterised in that** it comprises spring means (103) disposed between said needle-carrier (20) and said catheter body (10), said spring means being loaded when said first and second sleeve are in the forward use position and being released when said first and second sleeve are in the retracted safety position.

4. A safety device (100) according to claim 3, **characterised in that** said spring means comprise a coil spring (103) disposed around said guide needle (3), wherein one end of said coil spring abuts against an annular abutment surface (25) of said needle-carrier and the other end of said spiral spring abuts against a collar or annular seal (16) protruding radially inward into said catheter body (10).

5. A safety device (100) according to any one of the preceding claims, **characterised in that** provided at the rear end of said catheter body is at least one nib (18) which protrudes radially outward to engage in a respective longitudinal guide slot (113) in said first sleeve and at the rear end of said first sleeve (101) at least one nib (118, 118') is provided which protrudes radially outward to engage in a respective longitudinal guide slot (143) of said second sleeve.

6. A safety device (100) according to claim 5, **characterised in that** at the rear end of said catheter body there is provided a pair of nibs (18) which protrude radially outward in diametrically opposite positions to engage in a respective pair of guide slots (113) disposed longitudinally in diametrically opposite positions of said first sleeve and at the rear end of said first sleeve (101) there is provided a pair of nibs (118, 118') which protrude radially outward in diametrically opposite positions to engage in a pair of guide slots (143) disposed longitudinally in diametrically opposite positions of said second sleeve, wherein one (118) of said nibs of the first sleeve protrudes radially outward from said guide slot (143) of said second sleeve to be able to be operated manually by the user.

7. A safety device (100) according to claim 5 or 6, **characterised in that** said at least one longitudinal slot (113, 143) of the first sleeve and of the second sleeve have respectively a rear notch (115, 145) and a forward abutment and stopping surface (114, 144) so that said longitudinal slot (113, 143) has a substantially L-shaped configuration to engage in a bayonet coupling relationship with said nib (18, 118, 118') of said catheter body and of said first sleeve.

8. A safety device (100) according to claim 7, **characterised in that** said rear notch (115, 145) of said longitudinal slot (113, 143) of said first and second sleeve is defined by an oblique abutment surface (116, 146) able to abut against a respective abutment surface (19, 120) of the respective nib (18, 118, 118') of said catheter body and said first sleeve.

9. A safety device (100) according to any one of claims 5 to 8, **characterised in that** in said longitudinal slot (113, 143) of said first and second sleeve there is provided a respective flexible tongue (117, 147) which has a forward abutment surface (112, 142) disposed in front of the forward abutment surface (114, 144) of said longitudinal slot (113, 143).

10. A safety device (100) according to claim 9, **characterised in that** said flexible tongue (117, 147) in said longitudinal slot (113, 143) is obtained by means of a longitudinal notch (119, 149) in the body of said first and said second sleeve, respectively.

11. A catheter (1) comprising a body (10) with an axial channel (11) communicating with a sheath or cannula (12) and a guide needle that can be inserted, through said channel (11), into said sheath (12) to guide it during insertion into the patient's body for administration of medicinal fluids, **characterised in that** it comprises a safety device (100) according to any one of the preceding claims.
